# EUROPÄISCHE PATENTANMELDUNG

(11) **EP 4 744 690 A1**
(43) Veröffentlichungstag der Anmeldung: **20.05.2026**
(21) Anmeldenummer: 25215849.8
(22) Anmeldetag: 14.11.2025
(51) Int. Cl.: A61M 1/16, A61B 50/33

(54) **DIALYSEMASCHINE MIT EINER LÖSBAREN, WENDBAREN AUFLAGEPLATTE, AUFLAGEPLATTE, VERWENDUNG EINER AUFLAGEPLATTE IN EINER DIALYSEMASCHINE UND SYSTEM AUS EINER DIALYSEMASCHINE UND EINEM FLUIDBEHÄLTER**

(30) Priorität: 19.11.2024 DE 102024133958
(71) Anmelder: B. Braun Avitum AG, 34212 Melsungen (DE)
(72) Erfinder: BERTRAM, Rolf, 34286 Bergheim (DE)
(74) Vertreter: Winter, Brandl - Partnerschaft mbB

(57) **Zusammenfassung**

Die vorliegende Offenbarung betrifft eine Dialysemaschine (1) mit einer entnehmbaren Auflageplatte (6; 206; 306; 406) für einen Fluidbehälter (2), wobei die Auflageplatte (6; 206; 306; 406) eine erste Oberfläche (18) und eine zweite gegenüberliegende Oberfläche (20) aufweist, wobei die erste Oberfläche (18) zumindest einen Vorsprung (22) aufweist und die zweite gegenüberliegende Oberfläche (20) sich von der ersten Oberfläche (18) unterscheidet, bevorzugt im Wesentlichen planar/ flach ausgebildet ist. Der zumindest eine Vorsprung (22) ist dafür vorgesehen und ausgebildet, in eine entsprechende Ausnehmung (26) des Fluidbehälters (2) einzugreifen bzw. in dieser aufgenommen zu sein. Die Auflageplatte (6; 206; 306; 406) ist abhängig von der Form des Fluidbehälters (2) in einer ersten Einsetzausrichtung und einer zweiten Einsetzausrichtung in einen Aufnahmeabschnitt (4) der Dialysemaschine (1) eingesetzt. Die erste Oberfläche (18) zeigt in der ersten Einsetzausrichtung zu dem Aufnahmeabschnitt (4) und in der zweiten Einsetzausrichtung zeigt die zweite Oberfläche (20) zu dem Aufnahmeabschnitt (4).

## Beschreibung

Die vorliegende Erfindung betrifft eine Dialysemaschine mit einer Auflage für Fluidbehälter.

### Hintergrund der Offenbarung

Dialysierflüssigkeit bzw. Dialysierflüssigkeitskonzentrat für Dialysemaschinen kann bekanntermaßen in Fluidbehältern aufbewahrt werden, die an der Dialysemaschine befestigt sind oder auf oder neben der Dialysemaschine stehen. Dafür weisen bekannte Dialysemaschinen einen Sockel mit einer Auflage- oder Abstellfläche auf, auf der die Fluidbehälter mit Dialysierflüssigkeit bzw. Dialysierflüssigkeitskonzentrat abgestellt oder gelagert werden können. Die Dialysemaschinen können auf Rollen aufgesetzt sein und können somit zwischen unterschiedlichen Behandlungsräumen bewegt werden. Da die Fluidbehälter an der Dialysemaschine angebracht sind, können sie bequem mit der Dialysemaschine bewegt werden. Beim Verschieben der Dialysemaschine besteht aber die Gefahr, dass die Fluidbehälter durch Unebenheiten am Boden oder sonstige Erschütterungen umkippen. Daher ist es bekannt, die Fluidbehälter mit Riemen oder Schnallen an der Dialysemaschine zu fixieren. Das Fixieren ist aber ein zusätzlicher Handgriff, der von einem Benutzer der Dialysemaschine getätigt werden muss.

Fluidbehälter für Dialysierflüssigkeit bzw. Dialysierflüssigkeitskonzentrat sind beispielsweise aus der DE 10 2022 118 053 A1 bekannt.

Es ist ferner bekannt, dass Dialysemaschinen Halter haben, die an die Form zumindest bestimmter Fluidbehälter angepasst sind. Somit kann der Fluidbehälter einfach in den entsprechenden Halter eingesetzt werden. Ein Beispiel für einen solchen Halter für einen Fluidbehälter ist aus der EP 3 325 037 B1 bekannt. Dieses Prinzip ähnelt bekannten Getränkehaltern in Autos. Dabei passen die Halter aber meist nur für eine bestimmte Behälterform. Meist passen also nur Fluidbehälter, die vom gleichen Hersteller wie die Dialysemaschine vertrieben werden, in den Halter. In der Praxis sind aber eine Vielzahl an unterschiedlichen Fluidbehältern mit unterschiedlichen Formen am Markt erhältlich und in Krankenhäusern in Gebrauch.

### Kurzbeschreibung der Offenbarung

Die Aufgabe der vorliegenden Offenbarung liegt also darin, die Nachteile des Standes der Technik zu überwinden oder zumindest zu mindern und insbesondere darin, eine Dialysemaschine bereitzustellen, an der Fluidbehälter mit unterschiedlichen Formen oder Geometrien einfach und sicher befestigt werden können.

Diese Aufgabe wird offenbarungsgemäß durch eine Dialysemaschine nach Anspruch 1 gelöst. Ferner wird die Aufgabe der vorliegenden Offenbarung durch ein System nach Anspruch 14 gelöst. Die Aufgabe wird außerdem durch eine Verwendung einer Auflageplatte nach Anspruch 16 und eine Auflageplatte nach Anspruch 17 gelöst.

Die vorliegende Offenbarung betrifft eine extrakorporale Blutbehandlungsmaschine oder eine Dialysemaschine mit einer entnehmbaren/ lösbaren Auflageplatte für mindestens einen Fluidbehälter, insbesondere für einen Behälter für Dialysierflüssigkeit bzw. Dialysierflüssigkeitskonzentrat. Die Auflageplatte weist eine erste Oberfläche und eine zweite Oberfläche, die der ersten Oberfläche gegenüberliegt, auf. Die erste Oberfläche weist zumindest einen (konvexen) Vorsprung/ Dom bzw. eine (konvexe) Ausbuchtung auf. Der zumindest eine (konvexe) Vorsprung ist dafür vorgesehen und ausgebildet, in eine entsprechende (konkave) Ausnehmung/ Aussparung des Fluidbehälters einzugreifen bzw. in dieser aufgenommen zu sein. Die zweite gegenüberliegende Oberfläche unterscheidet sich von der ersten Oberfläche. Dabei kann die zweite gegenüberliegende Oberfläche beispielsweise (im Wesentlichen) plan/ planar/ flach ausgebildet sein. Die Auflageplatte ist abhängig von der Form des Fluidbehälters in einer ersten Einsetzausrichtung oder einer zweiten Einsetzausrichtung in einen Sockel/ Aufnahmeabschnitt der Dialysemaschine eingesetzt. In der ersten Einsetzausrichtung zeigt die erste Oberfläche in Richtung des Sockels/ Aufnahmeabschnitts und in der zweiten Einsetzausrichtung zeigt die zweite Oberfläche in Richtung des Sockels/ Aufnahmeabschnitts.

In anderen Worten ist die Auflageplatte derart abhängig von der Form/ Außenkontur des Fluidbehälters in den Aufnahmeabschnitt/ Sockel der Dialysemaschine eingesetzt, dass eine zur Form des Fluidbehälters passende Seite/ Oberfläche der Auflageplatte nach oben bzw. in Richtung des Fluidbehälters zeigt. D.h. die Auflageplatte kann in einer ersten Ausrichtung und in einer weiteren, zu der ersten Ausrichtung auf den Kopf gestellten Ausrichtung in den Sockel/ Aufnahmeabschnitt der Dialysemaschine eingesetzt zu werden.

In nochmals anderen Worten weist die Dialysemaschine also eine wendbare (Auflage-)Platte bzw. eine Wendeplatte auf, auf der der Fluidbehälter oder die Fluidbehälter abgestellt oder platziert werden können. Die Wendeplatte kann basierend auf der Form der Fluidbehälter in die entsprechende Richtung ausgerichtet werden und kann somit (zusammen mit dem Aufnahmeabschnitt/ Sockel) eine für den Fluidbehälter passende Auflagefläche/ Abstellfläche ausbilden.

Der zumindest eine (konvexe) Vorsprung kann dabei eine Ausbuchtung sein, die (im Wesentlichen senkrecht) aus der ersten Oberfläche hervorragt und somit als ein Befestigungselement funktioniert. Der (konvexe) Vorsprung kann also domförmig aus der ersten Oberfläche hervorstehen. D.h. das durch die Form des hervorstehenden domförmigen Vorsprungs eine Kuppel ausgebildet werden kann, die die Form eines Doms/ einer konvexen Wölbung aufweist. Der (konvexe) Vorsprung kann insbesondere rund/ rotationssymmetrisch ausgebildet sein. Der Vorsprung kann von seiner Form an die Ausnehmung des Fluidbehälters angepasst sein, sodass der Vorsprung mit der entsprechenden Ausnehmung zusammen eine Befestigung darstellt.

Die Auflageplatte kann im Wesentlichen als eine flache Platte mit der ersten Oberfläche und der zweiten Oberfläche, die der ersten Oberfläche gegenüberliegt, ausgebildet sein. Zwischen den beiden Oberflächen kann eine umlaufende Umfangsfläche ausgebildet sein. Die Auflageplatte kann selbstverständlich mehrere, insbesondere zwei Vorsprünge aufweisen. Auf jeden der mehren Vorsprünge kann dann ein eigener Fluidbehälter aufgesetzt werden.

Der Aufnahmeabschnitt/ Sockel kann als Teil eines Gehäuses der Dialysemaschine ausgebildet sein. Dabei kann der Aufnahmeabschnitt/ Sockel eine Auflage- oder Abstellfläche für die Fluidbehälter ausbilden, die von oben zugänglich ist. Der Aufnahmeabschnitt/ Sockel kann dabei auch wie ein Balkon aus dem Gehäuse hervorragen.

Bei dem Fluidbehälter kann es sich insbesondere um einen Fluidbehälter für Dialysierflüssigkeit bzw. Dialysierflüssigkeitskonzentrat handeln. Bei dem Fluid in dem Fluidbehälter kann es sich etwa um ein saures oder basisches Konzentrat handeln. Dialysierflüssigkeit kann aus Wasser hergestellt werden, das mit einer sauren Komponente und einer basischen Komponente gemischt wird. Bei der sauren Komponente kann es sich um eine Lösung mit Natriumchlorid, Kaliumchlorid, Magnesiumchlorid, Calciumchlorid, Glucose, Essigsäure und/oder Zitronensäure oder mit Acetat handeln. Die basische Komponente kann beispielsweise Natriumhydrogencarbonat oder (Bi-)Karbonat sein. Bei dem Fluidbehälter kann es sich alternativ um einen Behälter für ein Desinfektionsmittel handeln.

Der Fluidbehälter kann die Form einer (runden) Flasche oder die Form eines (eckigen) Kanisters aufweisen. Beiden Formen kann gemeinsam sein, dass der Fluidbehälter einen Bodenabschnitt aufweist, von dem eine einzelne umlaufende Seitenwand oder mehrere gegenüberliegende Seitenwände abstehen. Insbesondere eine (runde) Flasche kann einen (runden) Bodenabschnitt aufweisen, der eine Auflagefläche für den Kontakt mit der Auflageplatte und einen rückversetzten Abschnitt aufweist, der die konkave Ausnehmung ausbildet. Die Ausnehmung der Flasche ist dafür vorbereitet und ausgebildet, den (konvexen) Vorsprung aufzunehmen. Der Vorsprung kann dabei derart in die Ausnehmung eingreifen, dass beide Bauteile aneinander befestigt sind. Vorzugsweise kann eine Außenfläche des Vorsprungs an einer Innenfläche der Ausnehmung anliegen, sodass ein Reibschluss realisiert sein kann.

Der Kanister/ der Kanister-förmige Fluidbehälter kann einen im Wesentlichen flachen Bodenabschnitt aufweisen. D.h. eine bodenseitige Außenfläche des Fluidbehälters kann im Wesentlichen flach ausgebildet sein. Somit kann der Kanister eine große Auflagefläche auf der Auflageplatte haben.

Durch die offenbarungsgemäße Dialysemaschine können Fluidbehälter mit unterschiedlichen Formen oder Geometrien an der Dialysemaschine abgestellt und/oder fixiert werden. Abhängig von der Form des Fluidbehälters kann ein Benutzer die entsprechende passende Geometrie der Auflageplatte auswählen und die Auflageplatte so in die Dialysemaschine einsetzen, dass die passende Seite/ Geometrie nach oben/ zum Fluidbehälter zeigt. Wenn es sich bei dem Fluidbehälter um eine Flasche mit einer Ausnehmung im Bodenabschnitt handelt, kann der Benutzer die Auflageplatte derart in den Aufnahmeabschnitt/ Sockel einsetzten, dass die zweite Oberfläche in Richtung des Aufnahmeabschnitts/ Sockels und damit in Richtung des Bodens zeigt. Dadurch zeigt die gegenüberliegende erste Oberfläche nach oben bzw. in Richtung des Fluidbehälters und bildet damit eine Abstellfläche/ Ablagefläche/ Auflagefläche für den Fluidbehälter. Durch den zumindest einen (konvexen) Vorsprung bildet die Abstellfläche aber keine glatte/ flache/ plane Fläche aus, sondern eine (Abstell-)Fläche mit einem oder mehreren Befestigungselementen. Wenn der Fluidbehälter dann auf die Auflageplatte aufgesetzt wird, greift der (konvexe) Vorsprung der Auflageplatte in die entsprechende konkave Ausnehmung des Fluidbehälters auf der Auflageplatte, bzw. ist zumindest darin aufgenommen. Somit kann eine Außenfläche des Vorsprungs (reibschlüssig) an einer Innenfläche der Ausnehmung anliegen. Dieses Ineinandergreifen kann den Fluidbehälter auf der Auflageplatte und somit an der Dialysemaschine befestigen. Es kann offenbarungsgemäß jedoch auch vorgesehen sein, dass die Außenfläche des Vorsprungs nicht an der Innenfläche der Ausnehmung anliegt. In diesem Fall dient der Vorsprung der Auflageplatte eher als Positionierungshilfe für den Fluidbehälter, um den Fluidbehälter an einer geeigneten Stelle abzustellen, etwa derart, dass neben diesem Fluidbehälter noch ein weiterer Fluidbehälter auf der Auflageplatte abgestellt werden kann.

Der (konvexe) Vorsprung kann entsprechend zusätzlich oder alternativ zu der Befestigung bzw. Fixierung auch eine Positionierungshilfe sein bzw. als eine

Positionierungshilfe dienen. Dem Benutzer, der den Fluidbehälter auf der Auflageplatte abstellt, wird durch den Vorsprung angezeigt, wo die optimale Position für die Platzierung des Fluidbehälters ist. Dadurch kann sichergestellt werden, dass auch noch ein weiterer Fluidbehälter neben den ersten Fluidbehälter gestellt werden kann. Da die gefüllten Fluidbehälter schwer sein können, ist es vorteilhaft, wenn die Fluidbehälter von dem Benutzer möglich wenig hochgehoben werden müssen.

Wenn ein Fluidbehälter die Form eines Kanisters mit einem flachen Bodenabschnitt aufweist, dann kann der Benutzer die Auflageplatte derart in den Aufnahmeabschnitt/ Sockel einsetzen, dass die erste Oberfläche in Richtung des Aufnahmeabschnitts/ Sockels zeigt. Die zweite Oberfläche zeigt somit in die entgegengesetzte Richtung nach oben/ in Richtung des Fluidbehälters. D.h. die flache/ plane Oberfläche wird somit zu einer (im Wesentlichen ebenfalls planen) Abstellfläche/ Ablagefläche/ Auflagefläche für den Fluidbehälter. Dadurch kann der Kanister mit dem flachen Bodenabschnitt auf die flache/ plane Abstellfläche aufgestellt werden. Die flache/ plane Abstellfläche passt zu dem flachen Bodenabschnitt, sodass der Kanister sicher auf der Auflageplatte steht und auch bei Bewegung der Dialysemaschine nicht umkippt. Durch die flache/ plane Abstellfläche kann eine Universalaufnahme bereitgestellt werden, auf die Fluidbehälter oder Kanister mit verschiedenen Bodenabschnitten passen.

Die Aufgabe der vorliegenden Offenbarung wird ferner durch ein System aus der Dialysemaschine nach einem der vorstehenden Aspekte und zumindest einem Fluidbehälter gelöst.

Vorteilhafte Weiterentwicklungen der Offenbarung sind Gegenstand der beigefügten Unteransprüche.

Nach einem optionalen Aspekt der vorliegenden Offenbarung kann der Aufnahmeabschnitt/ Sockel eine Vertiefung/ Aussparung/ Aufnahme / einen Freiraum aufweisen, in die sich der (konvexe) Vorsprung erstreckt, wenn die Auflageplatte in der ersten Einsetzausrichtung eingesetzt ist. D.h. der Vorsprung kann von der Vertiefung aufgenommen werden. In anderen Worten weist der Aufnahmeabschnitt/ Sockel einen Auflageabschnitt für die Fluidbehälter und die (mittlere) Vertiefung auf. Die Vertiefung erstreckt sich von dem Auflageabschnitt in Richtung des Bodens/ in Richtung der Räder der Dialysemaschine. Da der Vorsprung der Auflageplatte in die Vertiefung eintauchen kann, liegt der Vorsprung nicht direkt auf einem Auflageabschnitt des Aufnahmeabschnitts/ Sockels auf und steht somit nicht aus dem Aufnahmeabschnitt/ Sockel hervor.

Vorzugsweise kann die Vertiefung an die Form der Auflageplatte angepasst sein. D.h. die Vertiefung kann derart geformt sein, dass die Auflageplatte zumindest teilweise in die Vertiefung eingesetzt werden kann.

Vorzugsweise kann der Aufnahmeabschnitt eine umlaufende Nut aufweisen, die die Vertiefung umgibt und wobei die Auflageplatte auf einem Nutabschluss der umlaufenden Nut aufliegen kann. In anderen Worten kann die Vertiefung eine umlaufende Auflagefläche aufweisen, auf der die Auflageplatte aufliegen kann. Die Auflagefläche kann dabei als Stufe in einer Seitenwand der Vertiefung ausgebildet sein. D.h. die Auflagefläche kann dabei von der Seitenwand der Vertiefung aus ins Innere der Vertiefung vorstehen und kann auch von dem Auflageabschnitt parallel (in Richtung eines Bodens der Vertiefung) beabstandet sein. Der Abstand zwischen der Auflagefläche und dem Auflageabschnitt kann dabei insbesondere der Dicke/ Höhe der Auflageplatte entsprechen. Dadurch kann die Auflageplatte bündig mit dem Auflageabschnitt abschließen und eine im Wesentlichen ebene Auflage- oder Abstellfläche für die Fluidbehälter ausbilden. Die Auflagefläche kann derart ausgebildet sein, dass die Auflageplatte und ein Boden der Vertiefung immer beabstandet sind. Dadurch kann Flüssigkeit, die von einem der Fluidbehälter ausgelaufen ist, immer abfließen.

Die Vertiefung mit der Auflagefläche, auf der die Auflageplatte aufliegt, können zusammen eine Fassung für die Auflageplatte ausbilden. D.h. die Auflageplatte kann in die Fassung eingesetzt werden und kann durch die Fassung gegen ein Verrutschen in seitliche Richtung, d.h. in Breitenrichtung, gesichert werden.

Nach einem weiteren optionalen Aspekt der vorliegenden Offenbarung kann ein Spalt zwischen der Auflagefläche bzw. der Fassung und der Auflageplatte ausgebildet sein. D.h. es kann ein Abstand zwischen der Auflagefläche und der Auflageplatte vorhanden sein. Der Spalt kann dabei sowohl in Längs- bzw. Breitenrichtung der Auflageplatte als auch in Höhen- bzw. Dickenrichtung der Auflageplatte ausgebildet sein. Der Spalt in Längs- bzw. Breitenrichtung der Auflageplatte kann derart ausgebildet sein, dass die Auflageplatte in einer Längs- und/oder Breitenerstreckung kleiner ist als die Fassung bzw. die Vertiefung, in der die Auflageplatte eingefasst ist. Dadurch kann ein Spalt zwischen einer Umlaufsfläche der Auflageplatte, die zwischen den zwei gegenüberliegenden Oberflächen ausgebildet ist, und der Fassung entstehen. Der Spalt in Höhen- bzw. Dickenrichtung der Auflageplatte kann beispielsweise durch Abstandshalter ausgebildet sein. Durch den Spalt kann Flüssigkeit, die aus einem der Fluidbehälter ausgelaufen ist oder verschüttet wurde, von der Auflageplatte abfließen. Die Flüssigkeit kann dabei insbesondere in die Vertiefung bzw. ins Innere des Aufnahmeabschnitts/ Sockels abfließen, wo die Flüssigkeit gesammelt wird.

Vorzugsweise ist der Boden der Vertiefung in Relation zur Horizontalen geneigt/ weißt einen Winkel zur Horizontalen auf, damit die Flüssigkeit in eine definierte Richtung fließt und sich an einem vorbestimmten Ort am Boden der Vertiefung sammelt.

Vorzugsweise kann der Spalt zwischen der Auflageplatte und der Auflagefläche durch Abstandshalter/ Auflagepins/ Distanzpins gebildet werden, die die Auflageplatte von der Auflagefläche beabstanden. Die Abstandshalter können dabei insbesondere senkrecht aus der ersten Oberfläche und/oder der zweiten Oberfläche der Auflageplatte hervorragen. Die Abstandshalter können einzelne Pins sein, die voneinander beabstandet sind. Die Abstandshalter können aber auch als dünne vorstehende Stege ausgebildet sein, die durch einzelne Durchlässe unterbrochen sind. Die Abstandshalter können bevorzugt in einem Randabschnitt der Auflageplatte angeordnet sein. Somit können die Abstandshalter auf der Auflagefläche aufliegen. Ferner können die Abstandshalter seitlich an der Fassung anliegen und die Auflageplatte gegen seitliches Verrutschen absichern.

Alternativ können die Abstandshalter/ Auflagepins/ Distanzpins auch an der Auflagefläche ausgebildet sein. Die Abstandshalter können dabei insbesondere nach oben bzw. in Richtung der Auflageplatte aus der Auflagefläche hervorstehen.

Vorzugsweise kann die Auflageplatte Öffnungen zur Handhabung aufweisen. Dabei kann die Auflageplatte eine Aussparung aufweisen, die sich von einer Oberfläche zur gegenüberliegenden Oberfläche durch die Dicke der Auflageplatte erstreckt. Dadurch kann der Benutzer mit den Fingern in die Auflageplatte greifen und die Auflageplatte einfach greifen, die bündig in der Fassung liegt.

Nach einem weiteren optionalen Aspekt der vorliegenden Offenbarung kann der zumindest eine (konvexe) Vorsprung eine Öffnung auf der zweiten Oberfläche ausbilden. Die Öffnung kann mit einem Deckel verschlossen sein. Da die Auflageplatte eine annähernde konstante Wandstärke/ Dicke hat, kann der (konvexe) Vorsprung auf der ersten Oberfläche eine entsprechende konkave Öffnung ausbilden. In der Öffnung kann sich Flüssigkeit oder Bakterien ansammeln und das Reinigen der Auflageplatte kann erschwert werden. Somit kann es vorteilhaft sein, die Öffnung durch den Deckel zu verschließen. Durch den Deckel kann die zweite Oberfläche im Wesentlichen eben/ plan ausgebildet sein und in der zweiten Einsetzausrichtung eine ebene Abstellfläche ausbilden.

Der Deckel kann dabei aus Kunststoff gefertigt sein und mit der Auflageplatte verschweißt sein. Dabei kann der Deckel insbesondere durch Laserschweißen an der Auflageplatte befestigt sein.

Die Öffnung kann auch ohne Deckel ausgebildet sein. Somit kann die zweite Oberfläche einen oder mehrere tiefe Ausbuchtungen aufweisen. In den Ausbuchtungen kann sich Flüssigkeit ansammeln, insbesondere wenn die Auflageplatte in der zweiten Einsetzausrichtung eingesetzt ist. Wenn die Öffnungen offengelassen werden, dann können Material- und Fertigungskosten für die Deckel bzw. für das Aufbringen der Deckel eingespart werden.

Vorzugsweise kann der zumindest eine Vorsprung eine Bohrung / eine Fluidauslassbohrung aufweisen, die insbesondere an einem Punkt des Vorsprungs positioniert ist, der am weitesten von der ersten Oberfläche beabstandet ist. Durch die Fluidauslassbohrung kann Flüssigkeit von der Auflageplatte in die Vertiefung abfließen.

Nach einem weiteren optionalen Aspekt der vorliegenden Offenbarung kann die Auflageplatte in Relation zur Horizontalen geneigt sein. Die Auflagefläche, auf dem die Auflageplatte in dem Aufnahmeabschnitt/ Sockel aufliegt, kann in einem Winkel relativ zur Horizontalen geneigt sein. Der Neigungswinkel kann beispielsweise zwischen 2° und 5°, bevorzugt 3° betragen. Durch die Neigung kann Flüssigkeit auf der Auflageplatte in eine definierte Richtung abfließen. Die Flüssigkeit kann sich somit in einer vorbestimmten Stelle/ Vertiefung/ Becken sammeln. Von dem vorbestimmten Sammelbecken kann die Flüssigkeit abgewischt oder abgesaugt werden. Die Auflagefläche des Aufnahmeabschnitts/ Sockels kann ebenfalls in Relation zur Horizontalen geneigt sein. Somit kann eine durchgängige Auflagefläche für die Fluidbehälter ausgebildet sein.

Vorzugsweise kann die Dialysemaschine einen Leckagesensor aufweisen, der an dem Aufnahmeabschnitt/ Sockel angebracht ist. Der Leckagesensor kann detektieren, wenn sich Flüssigkeit im Aufnahmeabschnitt/ Sockel ansammelt. Der Leckagesensor kann mit einer Steuereinheit verbunden sein, die eine Meldung über detektierte Flüssigkeit verarbeitet und an den Benutzer ausgibt. Der Leckagesensor kann beispielsweise an der tiefsten Stelle an dem Boden der Vertiefung angeordnet sein.

Alternativ kann die Auflageplatte einen Leckagesensor aufweisen. Der Leckagesensor kann detektieren, wenn sich Flüssigkeit auf der Auflageplatte ansammelt.

Nach einem optionalen Aspekt der vorliegenden Offenbarung kann die Auflageplatte lediglich einen Vorsprung aufweisen. Dadurch können zwei nebeneinanderliegende Stellplätze für unterschiedliche Fluidbehälter ausgebildet sein. Dabei könnte ein Abstellplatz für eine Flasche mit einer Ausnehmung in dem Bodenabschnitt ausgebildet sein und daneben ein Stellplatz für einen Kanister mit einem flachen Bodenabschnitt.

Die Auflageplatte kann auch jeweils einen Vorsprung an jeder der zwei Oberflächen ausbilden.

Vorzugsweise kann die Auflageplatte mit einem oder mehreren Magneten auf dem Aufnahmeabschnitt/ Sockel fixiert sein. Alternativ kann die Auflageplatte auch durch einen Formschluss oder eine andere lösbare Verbindung auf dem Aufnahmeabschnitt/ Sockel fixiert sein.

Der (konvexe) Vorsprung kann vorzugsweise lösbar an der Auflageplatte befestigt sein. Beispielsweise kann der Vorsprung geclipst oder geschraubt sein. Der Vorsprung kann auch durch ein anderes lösbares Fügeverfahren an der Auflageplatte befestigt sein.

Der Deckel kann auf die Auflageplatte aufgeschweißt, insbesondere durch Laserschweißen, werden. Alternativ kann der Deckel auch geklebt werden oder durch jedes andere stoffschlüssige Fügeverfahren an der Auflageplatte befestigt werden.

Die Auflageplatte kann beispielsweise durch Spritzguss gefertigt werden. Alternativ kann die Auflageplatte durch thermoplastischen Schaumguss (TSG), Reaction Injection Moulding (RIM) oder Tiefziehen gefertigt werden. Die Auflageplatte kann ferner 3D gedruckt werden. Die Auflageplatte kann auch durch ein Twin-Sheet-Verfahren gefertigt werden. Dabei können zwei Halbzeuge gleichzeitig tiefgezogen und dabei gefügt werden, sodass in einem einfachen Verfahren doppelwandige Bauteile geformt werden können.

Als Werkstoffe können für die Auflageplatte PE, PP oder PET oder andere schweißbare Kunststoffe, insbesondere thermoplastische Kunststoffe verwendet werden.

Ferner wird die Aufgabe der vorliegenden Offenbarung durch die Verwendung der Auflageplatte in oder an der Dialysemaschine, insbesondere eine Dialysemaschine wie voranstehend beschrieben, gelöst. Insbesondere kann die Auflageplatte als Unterlage/ Abstellfläche/ Ablage für den Fluidbehälter an der Dialysemaschine verwendet werden. Die Auflageplatte weist die erste Oberfläche und die zweite gegenüberliegende Oberfläche auf. Die erste Oberfläche weist den zumindest einen Vorsprung. Der zumindest eine Vorsprung ist dafür vorgesehen und ausgebildet, in die entsprechende Ausnehmung des Fluidbehälters einzugreifen oder darin aufgenommen zu werden. Die zweite gegenüberliegende Oberfläche unterscheidet sich von der ersten Oberfläche. Insbesondere kann die zweite gegenüberliegende Oberfläche flach oder plan ausgebildet sein. Die Auflageplatte wird abhängig von einer Form eines Bodenabschnitts des Fluidbehälters in der ersten Einsetzausrichtung oder der zweiten Einsetzausrichtung in den Aufnahmeabschnitt der Dialysemaschine eingesetzt bzw. ist in diesen einsetzbar. In der ersten Einsetzausrichtung zeigt die erste Oberfläche in Richtung des Aufnahmeabschnitts und in der zweiten Einsetzausrichtung zeigt die zweite Oberfläche in Richtung des Aufnahmeabschnitts.

Durch die offenbarungsgemäße Verwendung der Auflageplatte können Fluidbehälter mit unterschiedlichen Formen an der Dialysemaschine angeordnet/ befestigt bzw. sicher auf (dem Aufnahmeabschnitt) der Dialysemaschine abgestellt werden.

Ferner betrifft die vorliegende Offenbarung eine Auflageplatte für eine Dialysemaschine, insbesondere eine Dialysemaschine wie voranstehend beschrieben, mit der ersten Oberfläche und der zweiten gegenüberliegenden Oberfläche. Die erste Oberfläche weist den zumindest einen Vorsprung auf und die zweite gegenüberliegende Oberfläche unterscheidet sich von der ersten Oberfläche. Der zumindest eine Vorsprung ist dafür vorgesehen und ausgebildet ist, in die entsprechende Ausnehmung des Fluidbehälters einzugreifen oder darin aufgenommen zu werden. Die Auflageplatte kann dafür vorgesehen und ausgebildet sein, abhängig von einer Form eines Bodenabschnitts des Fluidbehälters in einer ersten Einsetzausrichtung oder einer zweiten Einsetzausrichtung in einen Aufnahmeabschnitt der Dialysemaschine eingesetzt zu werden. Je nach Einsetzrichtung kann eine der ersten und zweiten Oberflächen nach oben bzw. in Richtung des Aufnahmeabschnitts der Dialysemaschine zeigen.

Durch die offenbarungsgemäße Auflageplatte ist eine einfache und schnelle Änderung einer Aufnahmegeometrie an der Dialysemaschine möglich.

Vorzugsweise kann der zumindest eine Vorsprung die Öffnung auf der zweiten Oberfläche ausbilden. Vorzugsweise kann die Auflageplatte einen Deckel aufweisen, der die Öffnung verschließt. Dabei kann der Deckel auf der Öffnung verschweißt sein.

Vorteilhafterweise können die erste Oberfläche und/oder die zweite Oberfläche ferner vorstehende Abstandshalter aufweisen, die aus den jeweiligen Oberflächen konkav hervorstehen können.

Die Auflageplatte kann auch ohne die vorstehenden Abstandshalter ausgebildet sein. Das kann insofern vorteilhaft sein, da die Oberfläche der Auflageplatte dann flach/ plan ist und somit insbesondere den kanisterförmigen Fluidbehälter gut aufnehmen kann. In diesem Fall können die Abstandshalter vorteilhafterweise an der Fassung des Aufnahmeabschnitts der Dialysemaschine ausgebildet sein.

Insbesondere wenn die Auflageplatte keine Abstandshalter aufweist, kann es vorteilhaft sein, eine Geometrie vorzusehen, die ein Verrutschen der Auflageplatte verhindert. Dabei kann beispielsweise der Aufnahmeabschnitt eine oder mehrere Mulden aufweisen, in die ein oder mehrere entsprechende Vorsprünge an der Auflageplatte eingreifen können. Natürlich kann auch die Auflageplatte die eine Mulde oder die mehreren Mulden und der Aufnahmeabschnitt den einen Vorsprung oder die mehreren entsprechende Vorsprünge aufweisen Die Kombination von Mulde und Vorsprung kann dann ein ungewolltes Verrutschen der Auflageplatte relativ zu dem Aufnahmeabschnitt verhindern.

Es ist natürlich ebenfalls denkbar, dass die Auflageplatte nur an einer der beiden Oberflächen die Abstandshalter aufweist.

Der zumindest eine Vorsprung der Auflageplatte kann ferner eine Bohrung aufweisen, die insbesondere an einem Punkt des Vorsprungs positioniert sein kann, der am weitesten von der ersten Oberfläche beabstandet ist. Durch diese Bohrung kann Fluid aus der Öffnung abfließen, insbesondere wenn die Öffnung nicht durch einen Deckel verschlossen ist.

### Kurzbeschreibung der Figuren

Fig. 1 zeigt eine Dialysemaschine gemäß der vorliegenden Offenbarung;
Fig. 2 zeigt eine Auflageplatte einer Dialysemaschine gemäß einer ersten Ausführungsform der vorliegenden Offenbarung in einer ersten Einsetzausrichtung;
Fig. 3 zeigt einen Längsschnitt durch einen Ausschnitt der Dialysemaschine gemäß der ersten Ausführungsform der vorliegenden Offenbarung;
Fig. 4 zeigt die Auflageplatte der Dialysemaschine gemäß der ersten Ausführungsform der vorliegenden Offenbarung in einer zweiten Einsetzausrichtung;
Fig. 5 zeigt einen Ausschnitt der Dialysemaschine gemäß der ersten Ausführungsform der vorliegenden Offenbarung ohne Auflageplatte;
Fig. 6 zeigt eine erste Oberfläche der Auflageplatte der Dialysemaschine gemäß der ersten Ausführungsform der vorliegenden Offenbarung;
Fig. 7 zeigt eine zweite Oberfläche der Auflageplatte der Dialysemaschine gemäß der ersten Ausführungsform der vorliegenden Offenbarung;
Fig. 8 zeigt einen Sockel der Dialysemaschine gemäß der ersten Ausführungsform der vorliegenden Offenbarung mit zwei Fluidbehältern auf der Auflageplatte in der ersten Einsetzausrichtung;
Fig. 9 zeigt einen Sockel der Dialysemaschine gemäß der ersten Ausführungsform der vorliegenden Offenbarung mit zwei Fluidbehältern auf der Auflageplatte in der zweiten Einsetzausrichtung;
Fig. 10 zeigt eine Auflageplatte der Dialysemaschine gemäß der zweiten Ausführungsform der vorliegenden Offenbarung in der ersten Einsetzausrichtung;
Fig. 11 zeigt die Auflageplatte der Dialysemaschine gemäß der zweiten Ausführungsform der vorliegenden Offenbarung in der zweiten Einsetzausrichtung;
Fig. 12 zeigt eine Auflageplatte der Dialysemaschine gemäß einer dritten Ausführungsform der vorliegenden Offenbarung; und
Fig. 13 zeigt eine Auflageplatte der Dialysemaschine gemäß einer vierten Ausführungsform der vorliegenden Offenbarung.

### Detaillierte Beschreibung der Figuren

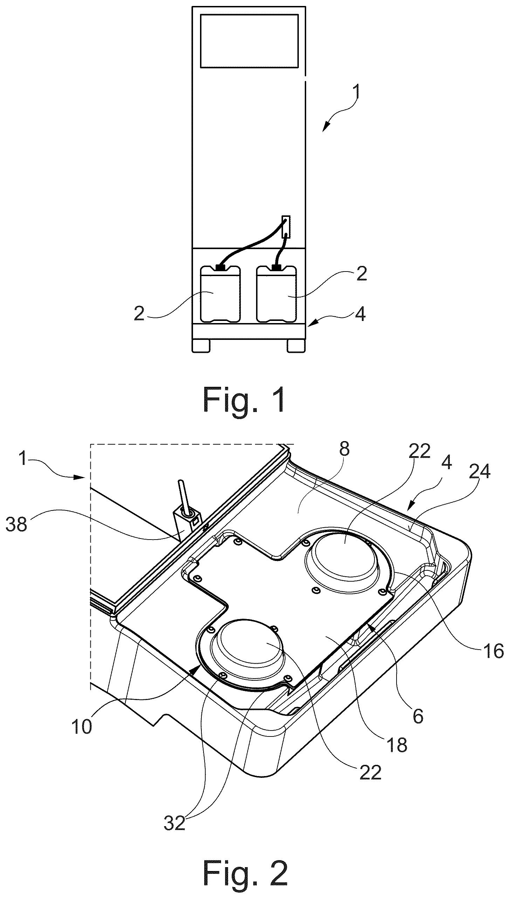

Fig. 1 zeigt eine Dialysemaschine 1 mit zwei Fluidbehältern 2. Die Dialysemaschine 1 hat Rollen an einer Unterseite der Dialysemaschine 1 und ist damit verschiebbar. Die Fluidbehälter 2 sind auf einem Aufnahmeabschnitt/ Abstellabschnitt/ Sockel 4 der Dialysemaschine 1 aufgestellt. Somit können die Fluidbehälter 2 mit der beweglichen Dialysemaschine 1 bewegt werden.

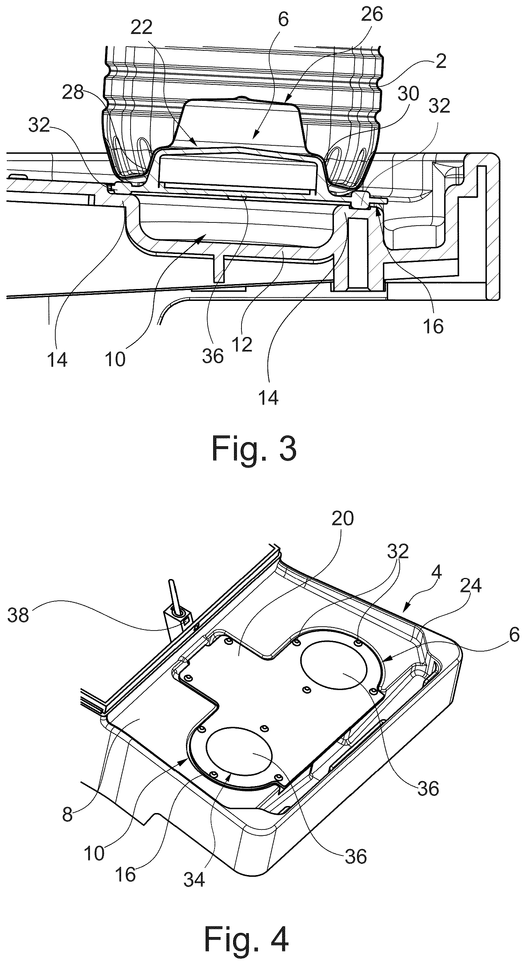

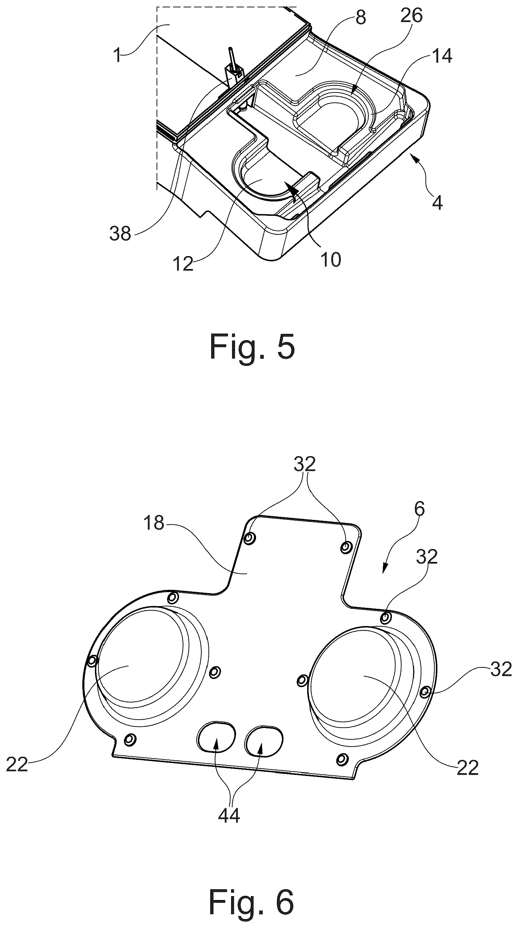

Fig. 2 zeigt eine Auflageplatte 6, die in einer ersten Einsetzausrichtung an oder in dem Sockel 4 aufliegt. Der Sockel 4 hat einen im Wesentlichen flachen Auflageabschnitt 8 für die Fluidbehälter 2 und eine mittige Fassung/ Aufnahme/ Freiraum/ Vertiefung 10 für die Auflageplatte 6. Die Vertiefung 10 hat im Wesentlichen die Form der Auflageplatte 6. D.h. eine Innenkontur der Vertiefung 10 ist an eine Außenkontur der Auflageplatte 6 angepasst. Ein Boden 12 der Vertiefung 10 (in Fig. 5 gezeigt) ist im Vergleich zu dem Auflageabschnitt 8 parallel nach unten/ in Richtung des Bodens der Dialysemaschine 1 versetzt. Somit bildet die Vertiefung 10 einen Freiraum in dem Sockel 4 aus. Die Vertiefung 10 hat eine Auflagefläche 14 (in Fig. 3 gezeigt), auf dem die Auflageplatte 6 aufliegt. Die Auflagefläche 14 ist von dem Auflageabschnitt 8 parallel beabstandet und ragt nach innen in die Vertiefung 10. Somit bildet die Auflagefläche 14 und die Innenkontur der Vertiefung eine Fassung für die Auflageplatte 6. Der Abstand zwischen der Auflagefläche 14 und dem Auflageabschnitt 8 entspricht im Wesentlichen der Dicke der Auflageplatte 6. Die Auflageplatte 6 liegt somit derart auf der Auflagefläche 14 auf, dass die Auflageplatte 6 und der Auflageabschnitt 8 bündig abschließen und eine gemeinsame Oberfläche/ Ebene ausbilden. Die Auflageplatte 6 bildet somit mit dem Auflageabschnitt 8 eine im Wesentlichen durchgehende Ablage- oder Abstelloberfläche für die Fluidbehälter 2 aus. Durch die Aufnahme in die Fassung, wird ein seitliches Verrutschen der Auflageplatte 6 verhindert. Eine Längs- oder Breitenerstreckung der Auflageplatte 6 ist kleiner als die Fassung/ Vertiefung 10. Dadurch entsteht ein Spalt 16 zwischen der Auflageplatte 8 und der Vertiefung 10. Durch den Spalt 16 kann Flüssigkeit von der Auflageplatte 8 in die Vertiefung 10 des Sockels 4 ablaufen und sich in der Vertiefung 10 sammeln.

Die Auflageplatte 6 hat eine erste Oberfläche 18 und eine zweite gegenüberliegende Oberfläche 20 (in Fig. 4 gezeigt). Die erste Oberfläche 18 hat zwei konvexe Vorsprünge 22, die aus der ersten Oberfläche 18 herausragen. In der ersten Einsetzausrichtung ist die Auflageplatte 6 derart ausgerichtet, dass die zweite Oberfläche 20 in Richtung des Sockels 4 zeigt. Somit zeigt die erste Oberfläche 18 von dem Sockel 4 weg nach oben und bildet eine Abstellfläche für die Fluidbehälter 2 aus. Der Auflageabschnitt 8 ist parallel zu einem Wandabschluss/ Rand 24 des Sockels 4 versetzt, sodass der Rand 24 über den Auflageabschnitt 8 herausragt. Somit kann Flüssigkeit, die aus den Fluidbehältern 2 ausläuft, in dem Sockel 4 aufgefangen werden.

Wenn die Fluidbehälter 2 auf dem Sockel 4 abgestellt werden, dann greifen die Vorsprünge 22 in entsprechende Ausnehmungen 26 an einem Bodenabschnitt des jeweiligen Fluidbehälters 2 ein. Somit kann der Fluidbehälter 2 in seiner Position fixiert oder gesichert werden. Dadurch wird ein Umfallen des Fluidbehälters 2 beim Bewegen der Dialysemaschine 1 verhindert. Ferner zeigen die Vorsprünge 22 die optimale Position für die einzelnen Fluidbehälter 2 an. Dadurch kann sichergestellt werden, dass zwei Fluidbehälter 2 nebeneinander auf dem Sockel 4 platziert werden können.

Fig. 3 zeigt einen Schnitt durch den Sockel 4 mit der Auflageplatte 6 in der ersten Einsetzausrichtung, wobei einer der Fluidbehälter 2 auf der Auflageplatte 6 steht. Der konvexe Vorsprung 22 greift in die Ausnehmung 26 des Fluidbehälters 2 ein. Die Ausnehmung 26 und der Vorsprung 22 sind dabei derart ausgebildet, dass eine Außenfläche 28 des Vorsprungs 22 an einer Innenfläche 30 der Ausnehmung 26 anliegt. Somit stützt der Vorsprung 22 den Fluidbehälter 2 ab und funktioniert somit als Befestigungselement.

Die Auflageplatte 6 weist Abstandshalter/ Abstandspins/ Distanzpins 32 auf, die im Wesentlichen senkrecht aus der zweiten Oberfläche 20 vorstehen. Wenn die Abstandshalter 32 auf der Auflagefläche 14 aufliegen, dann wird der Abstand/ Spalt 16 zwischen der Auflageplatte 6 und der Auflagefläche 14 ausgebildet. Durch diesen Spalt 16 kann verschüttete Flüssigkeit aus den Fluidbehältern 2 in die Vertiefung 10 des Sockels laufen. Die Abstandshalter 32 liegen auch an der Vertiefung 10 an und verhindern somit ein Verrutschen der Auflageplatte 6 in seitlicher Richtung.

Fig. 4 zeigt die Auflageplatte 6 in einer zweiten Einsetzausrichtung. In der zweiten Einsetzausrichtung zeigt die erste Oberfläche 18 in Richtung des Sockels 4 und die zweite Oberfläche 20 zeigt nach oben und bildet die Abstellfläche aus. Die zweite Oberfläche 20 ist plan/ flach ausgebildet. D.h. die plane zweite Oberfläche 20 und der bündige Auflageabschnitt 8 des Sockels 4 bilden zusammen eine im Wesentlichen ebene/ flache/ Abstellfläche oder Ablagefläche aus. Da die Auflageplatte 6 eine im Wesentlichen konstante Plattendicke hat, bilden die konvexen Vorsprünge 22 jeweils eine entsprechende (konkave) Öffnung/ Ausbeulung 34 in der zweiten Oberfläche 20 aus. Diese Öffnungen 34 sind jeweils durch Deckel 36 verschlossen, damit die zweite Oberfläche 20 flach ausgebildet ist und sich keine Flüssigkeit oder Bakterien in der Öffnung sammeln können. Die so gebildete flache Abstell- oder Ablagefläche ist ausgebildet, damit kanisterförmige Fluidbehälter 2 mit flachem Boden darauf abgestellt werden können. Die Kanister haben eine große Auflagefläche auf der Auflageplatte 6 und sind somit gut gegen Umkippen gesichert.

Zusammenfassend kann die Auflageplatte 6 also basierend auf der Form der Fluidbehälter 2 in der gewünschten/ passenden Einsetzausrichtung in den Sockel 4 eingesetzt werden. Somit zeigt immer die Seite/ Oberfläche nach oben, die am besten zur Form der Fluidbehälter 2 passt.

Fig. 5 zeigt den Sockel 4 ohne Auflageplatte. Der Sockel hat den Auflageabschnitt 8 und die Vertiefung 10, die mittig in dem Auflageabschnitt 8 ausgebildet ist. Die Vertiefung 10 ist an die Form der Auflageplatte 6 angepasst. Die Vertiefung 10 weist die Auflagefläche 14 auf, die von dem Auflageabschnitt 8 parallel beabstandet ist. Die Auflagefläche 14 und die Kontur der Vertiefung ergeben die Fassung für die Auflageplatte 8. Am tiefsten Punkt der Vertiefung 10 ist ein Leckagesensor 38 angeordnet. Der Leckagesensor 38 erfasst Flüssigkeit, die sich in der Vertiefung sammelt und gibt eine Warnung an eine Steuereinheit (nicht gezeigt) der Dialysemaschine 1 aus. Der Boden 12 der Vertiefung 10 ist relativ zur Horizontalen geneigt, damit sich Flüssigkeit an einem definierten Ort sammelt. Der Boden 12 ist dabei in Richtung der Dialysemaschine 1 geneigt. Die Auflagefläche 14 ist ebenfalls in Relation zur Horizontalen geneigt. Somit weist auch die Auflageplatte 6, die auf der Auflagefläche 14 aufliegt, einen Winkel bezüglich der Horizontalen auf. Dadurch kann Flüssigkeit von der Auflageplatte 6 abfließen. Die Flüssigkeit fließt bevorzugt durch den Spalt 16 zwischen der Auflageplatte 6 und der Fassung in die Vertiefung 10.

Fig. 6 zeigt die erste Oberfläche 18 der Auflageplatte 6. Die Auflageplatte 6 hat im Wesentlichen eine elliptische Grundform 40 mit einem Befestigungsabschnitt 42, der aus der elliptischen Grundform hervorsteht. Die beiden runden konvexen Vorsprünge 22 ragen aus der ersten Oberfläche 18 hervor. Die Auflageplatte 6 hat Handhabungsöffnungen 44 zur Handhabung der Auflageplatte 6. Die Auflageplatte 6 weist ferner eine Anzahl an vorstehenden Abstandshaltern 32 auf. Die Abstandshalter 32 liegen auf der Auflagefläche 14 auf und bilden somit den Spalt 16 bzw. einen Abstand zwischen der Auflagefläche 14 und der Auflageplatte 6, wenn die Auflageplatte 6 in den Sockel 4 eingesetzt ist. Durch diesen Spalt 16 kann verschüttete Flüssigkeit in die Vertiefung 10 in dem Sockel 4 ablaufen.

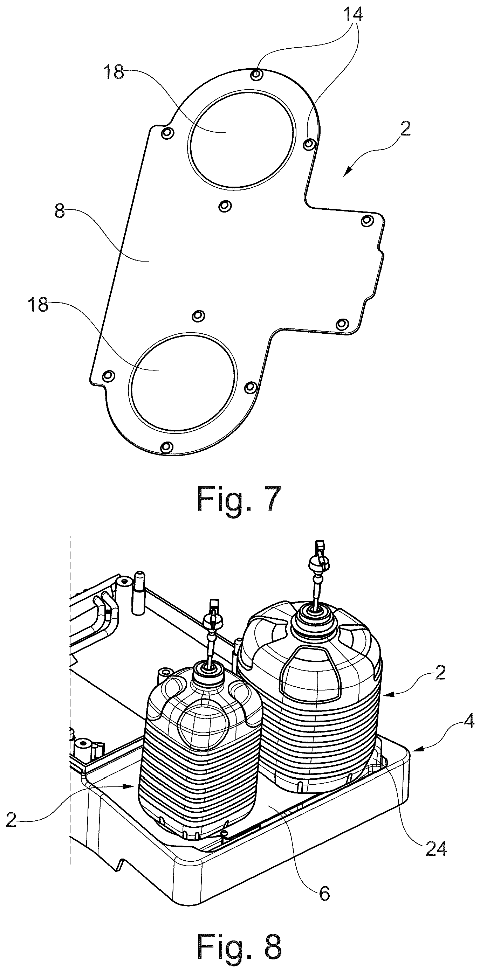

Fig. 7 zeigt die zweite Oberfläche 20 der Auflageplatte 6. Die Öffnungen 34, die von dem konvexen Vorsprung 22 auf der zweiten Oberfläche 20 ausgebildet sind, sind von den Deckeln 36 verschlossen. Die Deckel 36 können mit der Auflageplatte 36 verschweißt sein, insbesondere durch Laserschweißen. Auch die zweite Oberfläche 20 hat die Anzahl an Abstandshaltern 32, die im Wesentlichen senkrecht aus der zweiten Oberfläche 20 hervorstehen. Die Abstandshalter 32 sind jeweils um einen Umfang der Auflageplatte 6 verteilt.

Fig. 8 zeigt die zwei Fluidbehälter 2, die auf der Auflageplatte 6 aufgesetzt sind. Die Fluidbehälter 2 sind jeweils als Flaschen 46 ausgebildet und weisen eine Ausnehmung 26 auf. Deshalb ist die Auflageplatte 6 in der ersten Einsetzausrichtung ausgerichtet. D.h. die beiden konvexen Vorsprünge 22 zeigen nach oben in Richtung der Flaschen 46 und greifen jeweils in die Flaschen 46 ein. Da der Rand 24 des Sockels 4 über die Auflageplatte 6 hervorragt, kann der Rand 24 die Flaschen 46 zusätzlich abstützen. Die beiden Flaschen 46 haben ein unterschiedliches Volumen. Solange die Ausnehmungen 26 der Flaschen 46 zu den Vorsprüngen 22 passen, können die Flaschen 46 durch die Vorsprünge 22 befestigt werden.

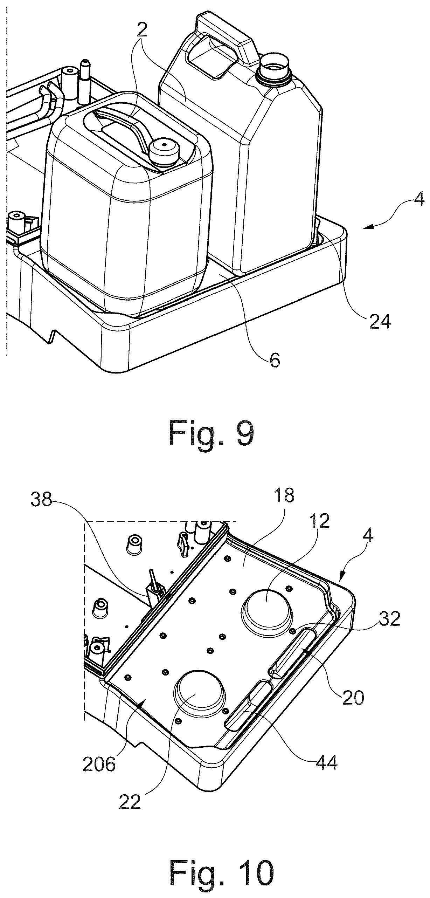

Fig. 9 zeigt zwei weitere Fluidbehälter 2, die auf der Auflageplatte 6 in der zweiten Einsetzausrichtung aufgestellt sind. Die Fluidbehälter sind als Kanister 48 geformt. Die Kanister 48 haben jeweils einen flachen Bodenabschnitt 50 von dem vier gegenüberliegende Seitenwände 52 hervorragen. Der flache Bodenabschnitt 50 kann auf der zweiten Oberfläche 20 der Auflageplatte 6 abgestellt werden. Durch den flachen Bodenabschnitt 50 haben die Kanister 48 eine derart große Auflagefläche, dass kein zusätzliches Befestigungselement notwendig ist. Die Fluidbehälter 2 sind zumindest abschnittsweise an dem überstehenden Rand 24 des Sockels 4 abgestützt.

Fig. 10 zeigt eine Auflageplatte 206 gemäß einer zweiten Ausführungsform. Die Unterschiede zwischen den Auflageplatten der einzelnen Ausführungsformen werden nachfolgend beschrieben. Ansonsten weist die Auflageplatte 206 nach der zweiten Ausführungsform alle Aspekte der Auflageplatte 6 nach der ersten Ausführungsform auf. Die Auflageplatte 206 ist annähernd rechteckig ausgebildet. Die Auflageplatte 206 gemäß der zweiten Ausführungsform ist größer als die Auflageplatte 6, die in den Figuren 2 bis 8 gezeigt ist. Die Auflageplatte 206 bedeckt den Auflageabschnitt 8 des Sockels 4 und ist von dem überstehenden Rand 24 des Sockels 4 eingefasst. Die Außenkontur der Auflageplatte ist also an die Innenkontur des Rands 24 angepasst. Der Rand 24 steht über die Auflageplatte 206 heraus. Somit kann keine Flüssigkeit aus dem Sockel 4 auslaufen. In Umfangsrichtung ist der Spalt 16 zwischen der Auflageplatte 206 und dem Rand 24 ausgebildet. Die erste Oberfläche 18 der Auflageplatte 206 weist die zwei konvexen Vorsprünge 22 auf. Die Auflageplatte 206 hat ferner die Handhabungsöffnungen 44 und die Anzahl an Abstandshaltern 32.

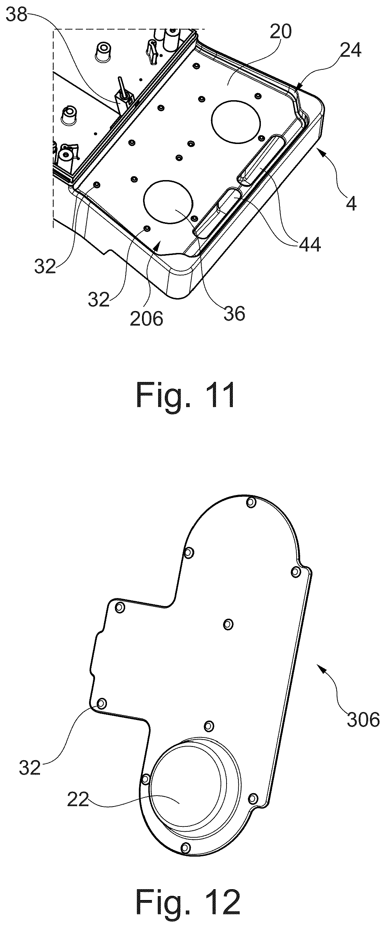

Fig. 11 zeigt die Auflageplatte 206 in der zweiten Einsetzausrichtung. Die Öffnungen 34 der zweiten Oberfläche 20 sind durch die Deckel 36 verschlossen. Somit ist die zweite Oberfläche 20 im Wesentlichen flach/ plan. Auch die zweite Oberfläche 20 weist die Abstandshalter 32 auf. Dadurch ist die Abstandshalter 32 ist die Auflageplatte 206 von dem Auflageabschnitt 8 des Sockels 4 beabstandet und Flüssigkeit kann von der Auflageplatte 206 in die Vertiefung 10 des Sockels 4 abfließen.

Selbstverständlich kann die Auflageplatte 6, 206 auch zwei Vorsprünge 22 aufweisen, die jeweils unterschiedliche Größen haben und dadurch für Ausnehmungen 26 mit unterschiedlichen Größen angepasst sind.

Fig. 12 zeigt eine Auflageplatte 306 gemäß einer dritten Ausführungsform. Die Auflageplatte 306 gemäß der dritten Ausführungsform entspricht im Wesentlichen der Auflageplatte 6 gemäß der ersten Ausführungsform. Als einzigen Unterschied weist die Auflageplatte 306 lediglich einen konvexen Vorsprung 22 auf. D.h. die erste Oberfläche 18 der Auflageplatte 306 weist zwei unterschiedliche Abstellplätze nebeneinander auf, wobei der eine Abstellplatz für eine Flasche 46 mit einer Ausnehmung 26 ausgebildet und der andere Abstellplatz für einen Kanister 48 mit einem flachen Bodenabschnitt 50 ausgebildet ist.

Natürlich kann auch die Auflageplatte 206 gemäß der zweiten Ausführungsform ebenfalls nur einen konvexen Vorsprung 22 aufweisen und somit auch zwei unterschiedliche Abstellplätze nebeneinander ausbilden.

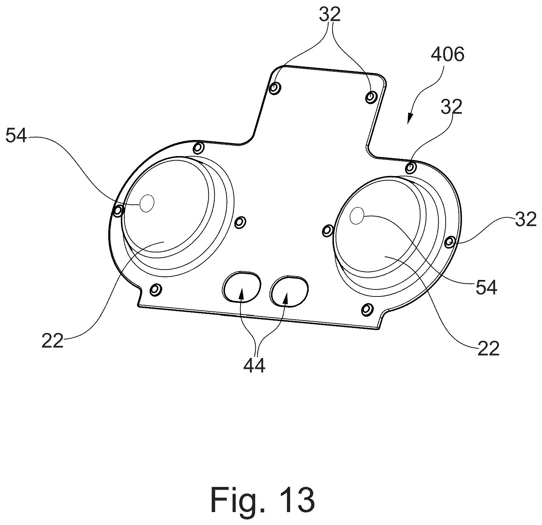

Fig. 13 zeigt eine Auflageplatte 406 gemäß einer vierten Ausführungsform. Die Auflageplatte 406 weist jeweils eine Bohrung/ Fluidauslassbohrung 54 in den konvexen Vorsprüngen 22 auf. Die Auflageplatte 406 hat vorzugsweise keine Deckel 36 auf den Öffnungen 34. D.h. in der zweiten Einsetzausrichtung kann sich Fluid in den (tiefen) Öffnungen 34 sammeln. Durch die Fluidauslassbohrungen 54 kann das Fluid von der Auflageplatte 406 in die Vertiefung 10 ablaufen. In weiteren Ausführungsformen kann die Auflageplatte 406 Handhabungsöffnungen 44 zur Handhabung der Auflageplatte 406 und/oder eine Anzahl an vorstehenden Abstandshaltern 32 aufweisen.

### Bezugszeichenliste

- 1: Dialysemaschine
- 2: Fluidbehälter
- 4: Sockel
- 6, 206, 306; 406: Auflageplatte
- 8: Auflageabschnitt
- 10: Vertiefung
- 12: Boden
- 14: Auflagefläche
- 16: Spalt
- 18: erste Oberfläche
- 20: zweite Oberfläche
- 22: konvexer Vorsprung
- 24: Rand
- 26: Ausnehmung
- 28: Außenfläche des Vorsprungs
- 30: Innenfläche der Ausnehmung
- 32: Abstandshalter
- 34: Öffnung
- 36: Deckel
- 38: Leckagesensor
- 44: Handhabungsöffnung
- 46: Flasche
- 48: Kanister
- 50: Bodenabschnitt
- 52: Seitenwand
- 54: Fluidauslassbohrung

## Patentansprüche

1. Dialysemaschine (1) mit einer entnehmbaren Auflageplatte (6; 206; 306; 406) für einen Fluidbehälter (2),
wobei die Auflageplatte (6; 206; 306; 406) eine erste Oberfläche (18) und eine zweite gegenüberliegende Oberfläche (20) aufweist,
wobei die erste Oberfläche (18) zumindest einen Vorsprung (22) aufweist,
wobei die zweite gegenüberliegende Oberfläche (20) sich von der ersten Oberfläche (18) unterscheidet,
wobei der zumindest eine Vorsprung (22) dafür vorgesehen und ausgebildet ist, in eine entsprechende Ausnehmung (26) des Fluidbehälters (2) einzugreifen oder darin aufgenommen zu sein,
wobei die Auflageplatte (6; 206; 306; 406) abhängig von einer Form eines Bodenabschnitts (50) des Fluidbehälters (2) in einer ersten Einsetzausrichtung oder einer zweiten Einsetzausrichtung in einen Aufnahmeabschnitt (4) der Dialysemaschine (1) eingesetzt oder einsetzbar ist, und
wobei in der ersten Einsetzausrichtung die erste Oberfläche (18) in Richtung des Aufnahmeabschnitts (4) zeigt und in der zweiten Einsetzausrichtung die zweite Oberfläche (20) in Richtung des Aufnahmeabschnitts (4) zeigt.

2. Dialysemaschine (1) nach Anspruch 1, **dadurch gekennzeichnet, dass** der Aufnahmeabschnitt (4) eine Vertiefung (10) aufweist, in die sich der zumindest eine Vorsprung (22) erstreckt, wenn die Auflageplatte (6; 206; 306; 406) in der ersten Einsetzausrichtung eingesetzt ist.

3. Dialysemaschine (1) nach Anspruch 2, **dadurch gekennzeichnet, dass** in der Vertiefung (10) eine umlaufende Auflagefläche (14) ausgebildet ist, auf der die Auflageplatte (6; 206; 306; 406) aufliegt.

4. Dialysemaschine (1) nach Anspruch 2, **dadurch gekennzeichnet, dass** ein Spalt (16) zwischen der Auflagefläche (14) und der Auflageplatte (6; 206; 306; 406) ausgebildet ist.

5. Dialysemaschine (1) nach Anspruch 4, **dadurch gekennzeichnet, dass** die Auflageplatte (6; 206; 306; 406) vorstehende Abstandshalter (32) aufweist, die die Auflageplatte (6; 206; 306; 406) von der Auflagefläche (14) beabstanden und den Spalt (16) bilden.

6. Dialysemaschine (1) nach Anspruch 4, **dadurch gekennzeichnet, dass** die Auflagefläche (14) vorstehende Abstandshalter (32) aufweist, die die Auflageplatte (6; 206; 306; 406) von der Auflagefläche (14) beabstanden und den Spalt (16) bilden.

7. Dialysemaschine (1) nach einem der vorstehenden Ansprüche, **dadurch gekennzeichnet, dass** der zumindest eine Vorsprung (22) eine Öffnung (34) auf der zweiten Oberfläche (20) ausbildet.

8. Dialysemaschine (1) nach Anspruch 7, **dadurch gekennzeichnet, dass** die Öffnung (34) mit einem Deckel (36) verschlossen, insbesondere verschweißt, ist.

9. Dialysemaschine (1) nach Anspruch 7, **dadurch gekennzeichnet, dass** der zumindest eine Vorsprung (22) eine Bohrung (54) aufweist, die insbesondere an einem Punkt des Vorsprungs (22) positioniert ist, der am weitesten von der ersten Oberfläche (18) beabstandet ist.

10. Dialysemaschine (1) nach einem der vorstehenden Ansprüche, **dadurch gekennzeichnet, dass** die Auflageplatte (6; 206; 306; 406), insbesondere im an dem Aufnahmeabschnitt (4) der Dialysemaschine (1) montierten Zustand, in Relation zur Horizontalen geneigt ist.

11. Dialysemaschine (1) nach einem der vorstehenden Ansprüche,
**gekennzeichnet durch** einen Leckagesensor (38), der an dem Aufnahmeabschnitt (4) angebracht ist, insbesondere an einem tiefsten Punkt eines Bodens (12) der Vertiefung (10).

12. Dialysemaschine (1) nach einem der vorstehenden Ansprüche, **dadurch gekennzeichnet, dass** der Boden (12) der Vertiefung (10) in Relation zur Horizontalen geneigt ist.

13. Dialysemaschine (1) nach einem der vorstehenden Ansprüche, **dadurch gekennzeichnet, dass** die Auflageplatte (6; 206; 306; 406) relativ zu einem Rand (24) des Sockels versetzt ist, sodass der Rand (24) über die Auflageplatte (6; 206; 306; 406) vorsteht.

14. Dialysemaschine (1) nach einem der vorstehenden Ansprüche, **dadurch gekennzeichnet, dass** die Auflageplatte (6; 206; 306; 406) und/oder der Aufnahmeabschnitt (4) durch Spritzguss oder Tiefziehen gefertigt ist.

15. System aus einer Dialysemaschine (1) nach einem der Ansprüche 1 bis 14 und einem Fluidbehälter (2) mit einem Bodenabschnitt (50) und einer Ausnehmung (26) in dem Bodenabschnitt (50), wobei der zumindest eine Vorsprung (22) in die entsprechende Ausnehmung (26) des Fluidbehälters (2) eingreift oder in dieser aufgenommen ist.

16. Verwendung einer Auflageplatte (6; 206; 306; 406) in einer Dialysemaschine (1) als Ablage für einen Fluidbehälter (2),
wobei die Auflageplatte (6; 206; 306; 406) eine erste Oberfläche (18) und eine zweite gegenüberliegende Oberfläche (20) aufweist,
wobei die erste Oberfläche (18) zumindest einen Vorsprung (22) aufweist,
wobei die zweite gegenüberliegende Oberfläche (20) sich von der ersten Oberfläche (18) unterscheidet,
wobei der zumindest eine Vorsprung (22) dafür vorgesehen und ausgebildet ist, in eine entsprechende Ausnehmung (26) des Fluidbehälters (2) einzugreifen oder darin aufgenommen zu sein,
wobei die Auflageplatte (6; 206; 306; 406) abhängig von einer Form eines Bodenabschnitts (50) des Fluidbehälters (2) in einer ersten Einsetzausrichtung oder einer zweiten Einsetzausrichtung in einen Aufnahmeabschnitt (4) der Dialysemaschine (1) eingesetzt wird oder einsetzbar ist, und
wobei in der ersten Einsetzausrichtung die erste Oberfläche (18) in Richtung des Aufnahmeabschnitts (4) zeigt und in der zweiten Einsetzausrichtung die zweite Oberfläche (20) in Richtung des Aufnahmeabschnitts (4) zeigt.

17. Auflageplatte (6; 206; 306; 406) für eine Dialysemaschine (1) zur Ablage eines Fluidbehälters (2), mit:
einer ersten Oberfläche (18) und einer zweiten gegenüberliegenden Oberfläche (20),
wobei die erste Oberfläche (18) zumindest einen Vorsprung (22) aufweist,
wobei die zweite gegenüberliegende Oberfläche (20) sich von der ersten Oberfläche (18) unterscheidet,
wobei der zumindest eine Vorsprung (22) dafür vorgesehen und ausgebildet ist, in eine entsprechende Ausnehmung (26) des Fluidbehälters (2) einzugreifen oder darin aufgenommen zu sein,
wobei die Auflageplatte (6; 206; 306; 406) abhängig von einer Form eines Bodenabschnitts (50) des Fluidbehälters (2) in einer ersten Einsetzausrichtung oder einer zweiten Einsetzausrichtung in einen Aufnahmeabschnitt (4) der Dialysemaschine (1) eingesetzt wird oder einsetzbar ist, und
wobei in der ersten Einsetzausrichtung die erste Oberfläche (18) in Richtung des Aufnahmeabschnitts (4) zeigt und in der zweiten Einsetzausrichtung die zweite Oberfläche (20) in Richtung des Aufnahmeabschnitts (4) zeigt.
